# EUROPÄISCHE PATENTSCHRIFT

(11) **EP 2 281 598 B1**
(45) Veröffentlichungstag und Bekanntmachung des Hinweises auf die Patenterteilung: **22.05.2019**
(21) Anmeldenummer: 09167232.9
(22) Anmeldetag: 05.08.2009
(51) Int. Cl.: A61M 16/18, A61M 16/01

(54) **Anästhesiesystem**
Anaesthetic system
Système d'anesthésie

(43) Veröffentlichungstag der Anmeldung: 09.02.2011
(73) Patentinhaber: Drägerwerk AG & Co. KGaA, 23558 Lübeck (DE)
(72) Erfinder: Faber, Sönke, 23617 Stockelsdorf (DE); Falb, Wolfgang, 23627 Groß Sarau (DE); Heidschmidt, Michael, 23558 Lübeck (DE); Heyer, Sven, 23558 Lübeck (DE); Kullik, Götz, 23568 Lübeck (DE); Mecklenburg, Frank, 23566 Lübeck (DE); Meyer, Martin, 23566 Lübeck (DE); Peter, Gerd, 23562 Lübeck (DE); Stark, Hartmut, 23617 Stockelsdorf (DE); Radomski, Klaus, 23566 Lübeck (DE)

(56) Entgegenhaltungen:
- EP-A- 1 563 859
- EP-B- 0 338 518
- DE-B3-102007 014 838
- DE-U1- 29 614 491
- US-A- 5 293 865

## Beschreibung

Die vorliegende Erfindung betrifft ein Anästhesiesystem gemäß dem Oberbegriff des Anspruches 1.

In der Medizintechnik werden für die Narkose von Patienten insbesondere bei chirurgischen Eingriffen überwiegend dampfförmige Anästhesiemittel eingesetzt. Hierfür ist ein Anästhesiesystem mit einem Anästhesiegerät und einem Anästhesiemitteldosierer erforderlich. In dem Anästhesiemitteldosierer mit einem Anästhesiemittel-Vorratsbehälter befindet sich das Anästhesiemittel. Bei aktiven Anästhesiemitteldosierern wird eine Energie verbrauchende elektrische Heizeinrichtung in dem Anästhesiemitteldosierer unter anderem zur Steuerung der Menge des Anästhesiemittels, welches dem Atemgas zugeführt wird, eingesetzt. Bei passiven Anästhesiemitteldosierern ist keine Energie verbrauchende elektrische Heizeinrichtung in dem Anästhesiemitteldosierer vorhanden. Die passiven Anästhesiemitteldosierer sind in der Herstellung preiswerter.

Die EP 0 338 518 B1 zeigt eine Narkoseeinrichtung mit einem Narkosemittelverdunster, aus dem das Narkosemittel einer Einstellvorrichtung zuführbar ist, von welcher aus dosierte Narkosemittelmengen an ein Narkosegerät abgebbar sind.

Die DE 10 2007 014 838 B3 zeigt ein gattungsbildendes Anästhesiesystem bestehend aus einem Anästhesiegerät, einem Anästhesiemitteldosierer mit einem Anästhesiemittel-Vorratsbehälter, wenigstens einer Dosierparametererfassungseinrichtung und einer kontaktlosen Schnittstelle zwischen dem Anästhesiegerät und dem Anästhesiemitteldosierer zur Übertragung von Daten, insbesondere der Dosierparameter und zur Energieversorgung der wenigstens einen Dosierparametererfassungseinrichtung, wobei die Daten und Energieübertragung durch elektromagnetische Feldkräfte erfolgt. In nachteiliger Weise ist die Übertragung von Daten und Energie durch elektromagnetische Feldkräfte technisch aufwendig. Ferner kann es bei der Verwendung von mehreren Anästhesiemitteldosierern an einem Anästhesiegerät zu gegenseitigen Störungen zwischen den Anästhesiemitteldosierern mit den jeweiligen Schnittstellen aufgrund der elektromagnetischen Feldkräfte kommen.

Die Aufgabe der vorliegenden Erfindung besteht darin, ein Anästhesiesystem zur Verfügung zu stellen, bei dem ein Füllstand an Anästhesiemittel in einem Anästhesiemitteldosierer und weitere Parameter des Anästhesiemitteldosierers einfach ermittelt und übertragen werden können. Ferner soll das Anästhesiesystem technisch einfach und zuverlässig im Betrieb arbeiten.

Die Lösung der Aufgabe erhält man für das Anästhesiesystem mit den Merkmalen von Anspruch 1.

Die Unteransprüche zu Anspruch 1 geben vorteilhafte Weiterbildungen des Anästhesiesystems nach Anspruch 1 an.

Die pneumatische Verbindung des wenigstens eine Anästhesiemitteldosierers mit dem Anästhesiegerät erfolgt vorzugsweise mit wenigstens einer pneumatischen Schnittstelle. Eine passive Dosierer-Schnittstelleneinheit verbraucht beim oder für den Betrieb keine Energie bzw. Hilfsenergie. Dadurch werden in vorteilhafter Weise im Anästhesiesystem keine Mittel zur Übertragung von Energie, insbesondere elektrischer Energie von dem Anästhesiegerät zu dem Anästhesiemitteldosierer benötigt, um mit dieser übertragenen Energie die Dosierer-Schnittstelleneinheit und/oder die Einrichtung mit Energie zum Betrieb zu versorgen. Zwar kann von dem Anästhesiegerät zu dem Anästhesiemitteldosierer Energie, beispielsweise Lichtenergie, übertragen werden. Diese Energie, beispielsweise Lichtenergie, wird jedoch nicht zum Betrieb der wenigstens einen Dosierer-Schnittstelleneinheit benötigt, sondern dient dazu, mittels des Lichtes Parameter des Anästhesiemitteldosierers zu erfassen. Die Energie ist somit lediglich ein, insbesondere unmittelbares und nicht gewandeltes, Parametererfassungsmittel.

In einer weiteren Ausgestaltung ist die Übermittlung von Daten von dem wenigstens einen Anästhesiemitteldosierer an das Anästhesiegerät mittels Infrarotstrahlung oder sichtbarem Licht oder UV-Strahlung ausführbar.

Infrarotstrahlung weist eine Wellenlänge zwischen 780 und 1 000 000 nm, insbesondere zwischen 780 und 5 000 nm auf. Sichtbares Licht weist eine Wellenlänge zwischen 380 und 780 nm auf. UV-Strahlung weist eine Wellenlänge zwischen 1 und 380 nm auf.

Die Kamera arbeitet im sichtbaren Spektralbereich oder infraroten Bereich oder im UV-Bereich.

In einer Variante umfasst die wenigstens eine Dosierer-Schnittstelleneinheit eine Codierung. In vorteilhafter Weise ist die Codierung eine optische Codierung.

In einer weiteren Ausführungsform ist die optische Codierung ein Strichcode und/oder ein zweidimensionaler Code, z. B. ein Datamatrixcode. Der Strichcode ist beispielsweise ein Kettencode mit einem einstückigen Strichcode von 12 bzw. 13 Bit-Daten. Der Code kann darüber hinaus mit einer Hammingdistanz größer als 2 versehen werden, so dass dadurch auch die Möglichkeit der Korrektur einzelner Lesefehler besteht. Dadurch können Lesefehler, beispielsweise resultierend aus einer Verschmutzung, ausgeglichen werden.

Insbesondere umfasst die wenigstens eine Geräte-Schnittstelleneinheit eine optische Encodereinheit zur optischen Erfassung der optischen Codierung der Dosierer-Schnittstelleneinheit. Vorzugsweise ist die optische Encodereinheit aus einer LED und der Kamera aufgebaut. Alternativ kann anstatt der LED auch eine Lampe vorgesehen sein. Die Kamera kann als eine CMOS-Chipkamera ausgebildet sein.

Erfindungsgemäß ist mit der wenigstens einen Parametererfassungseinrichtung ein Füllstand an Anästhesiemittel des Anästhesiemitteldosierers an dem Anästhesiegerät erfassbar.

In einer weiteren Ausgestaltung ist von der Einrichtung zur Erfassung des Füllstandes an Anästhesiemittel der Füllstand mittels einer Veränderung eines Brechungsindexunterschiedes ermittelbar, so dass bei einem unterschiedlichen Füllstand ein unterschiedlicher Brechungsindex und somit eine unterschiedliche Strahlstärke, insbesondere Lichtstärke an dem Empfänger auftritt. Die Erfassung des Füllstandes mittels einer Veränderung des Brechungsindex an einer optischen Grenzfläche stellt keine Energie verbrauchende oder Energie umwandelnde Einrichtung für den Betrieb der Einrichtung dar. Die elektromagnetische Strahlung wird für den Betrieb der Einrichtung nicht verbraucht.

Insbesondere ist von dem optischen Encoder der Geräte-Schnittstelleneinheit elektromagnetische Strahlung, insbesondere Licht, in einen Leiter, insbesondere Lichtleiter, der Einrichtung zur Erfassung des Füllstandes einleitbar. Die eingeleitete elektromagnetische Strahlung, insbesondere Licht, ist an einer optischen Grenzfläche in Abhängigkeit von dem Füllstand des Anästhesiemittels reflektierbar, wobei das wenigstens eine Bildmuster aus dem Unterschied der Strahlstärke der elektromagnetischen Strahlung erzeugt wird. Von der Kamera ist anhand der Strahlstärke, insbesondere der Lichtstärke, der reflektierten elektromagnetischen Strahlung, insbesondere Licht, der Füllstand erfassbar. Als Leiter für Licht oder elektromagnetische Strahlung kann beispielsweise ein Stab aus Glas oder Kunststoff oder auch eine oder mehrere Glasfasern verwendet werden.

In einer weiteren Ausgestaltung ist mit der wenigstens einen Parametererfassungseinrichtung eine Stellung, insbesondere Winkelstellung, einer Stelleinrichtung, insbesondere eines Handrades des Anästhesiemitteldosierers an dem Anästhesiegerät erfassbar. Die Stelleinrichtung umfasst eine Codierung, die als Bildmuster von der Kamera der wenigstens einen Geräte-Schnittstelleneinheit auslesbar ist. Mittels der Stelleinrichtung wird die Menge an Anästhesiemittel gesteuert und/oder geregelt, die dem durch den Anästhesiemitteldosierer geleiteten Atemgas zugeführt wird. Die Codierung an der Stelleinrichtung ist dabei dahingehend ausgebildet, dass die Codierung Daten enthält, aus denen die Stellung der Stelleinrichtung ermittelt werden kann. Die Codierung der Stelleinrichtung ist dabei eine optische Codierung, beispielsweise ein Strichcode. Alternativ hierzu kann auch ein zweidimensionaler Code oder eine Farbcodierung oder eine analoge Codierung mittels Erfassung einer Keilbreite vorgesehen sein.

In einer ergänzenden Ausführungsform sind von der Schnittstelle Daten bezüglich der Art des Anästhesiemitteldosierers und/oder einer Sachnummer des Anästhesiemitteldosierers und/oder einer Seriennummer des Anästhesiemitteldosierers und/oder der Art des Anästhesiemittels übertragbar und insbesondere sind diese Daten in dem wenigstens einen Bildmuster der Dosierer-Schnittstelleneinheit codiert gespeichert.

Aus der Art des Anästhesiemitteldosierers geht im Allgemeinen die Art des Anästhesiemittels hervor, weil für jede Art von Anästhesiemittel nur eine bestimmte Art eines Anästhesiemitteldosierers verwendet wird. Dadurch erhält das Anästhesiegerät die Information zur Art des verwendeten Anästhesiemittels.

Aus der Sachnummer geht die Version bzw. der Bauarttyp des Anästhesiemitteldosierers hervor. Dadurch erhält das Anästhesiegerät Daten hinsichtlich der konstruktiven und technischen Ausbildung des Anästhesiemitteldosierers.

Die Seriennummer des Anästhesiemitteldosierers ermöglicht es, den Anästhesiemitteldosierer zu identifizieren. Die Anästhesiemitteldosierer werden in einem Krankenhaus an unterschiedlichen Anästhesiegeräten genutzt. Bei einer erforderlichen Wartung eines Anästhesiemitteldosierers kann somit mit einem Netzwerk innerhalb eines Krankenhauses ein bestimmter Anästhesiemitteldosierer lokalisiert und identifiziert werden, so dass dadurch für eine Wartung oder eine Reparatur der Anästhesiemitteldosierer nicht aufwendig gesucht werden muss. Ferner kann in einer Seriennummer das Herstelldatum des Anästhesiemitteldosierers direkt oder indirekt beinhaltet sein. Alternativ dazu kann das Herstelldatum separat gespeichert sein.

In einer weiteren Ausgestaltung umfasst das Anästhesiesystem wenigstens zwei Anästhesiemitteldosierer und/oder das Anästhesiegerät ist dahingehend ausgebildet, dass wenigstens zwei Anästhesiemitteldosierer anschließbar sind, so dass beispielsweise zwei pneumatische Schnittstellen, zwei mechanische Befestigungseinrichtungen und zwei optische Encoder vorhanden sind. Bei diesem Anästhesiesystem ist es erforderlich, dass Vorrichtungen vorhanden sind die ein gleichzeitiges Verwenden von zwei Anästhesiemitteldosierern verhindern, damit nicht zwei unterschiedliche Anästhesiemittel dem Patienten zugeführt werden. Dies kann entweder dadurch ausgeführt werden, dass entweder eine Warneinrichtung beim gleichzeitigen Betrieb von zwei Anästhesiemitteldosierern vorhanden ist oder konstruktiv und technisch der gleichzeitige Betrieb von zwei Anästhesiemitteldosierern ausgeschlossen wird.

In einem passiven Anästhesiemitteldosierer, welcher über keine elektrische Heizeinrichtung zum Erwärmen des Anästhesiemittels verfügt, werden als Anästhesiemittel beispielweise Halothan, Isofluran, Sevofluran und Enfluran eingesetzt. Bei aktiven Anästhesiemitteldosierern mit einer elektrischen Heizung zum Erwärmen oder Temperieren des Anästhesiemittels auf eine bestimmte Temperatur wird beispielsweise Desfluran als Anästhesiemittel eingesetzt.

Im Nachfolgenden wird ein Ausführungsbeispiel der Erfindung unter Bezugnahme auf die beigefügten Zeichnungen näher beschrieben. Es zeigt:
- Fig. 1: eine Darstellung des erfindungsgemäßen Anästhesiesystems in einer ersten Ausführung,
- Fig. 2: einen vereinfachten Längsschnitt einer Einrichtung zur Erfassung eines Füllstandes eines Anästhesiemittels des Anästhesiesystems gemäß Fig. 1 und
- Fig. 3: eine Darstellung des erfindungsgemäßen Anästhesiesystems in einer zweiten Ausführung.

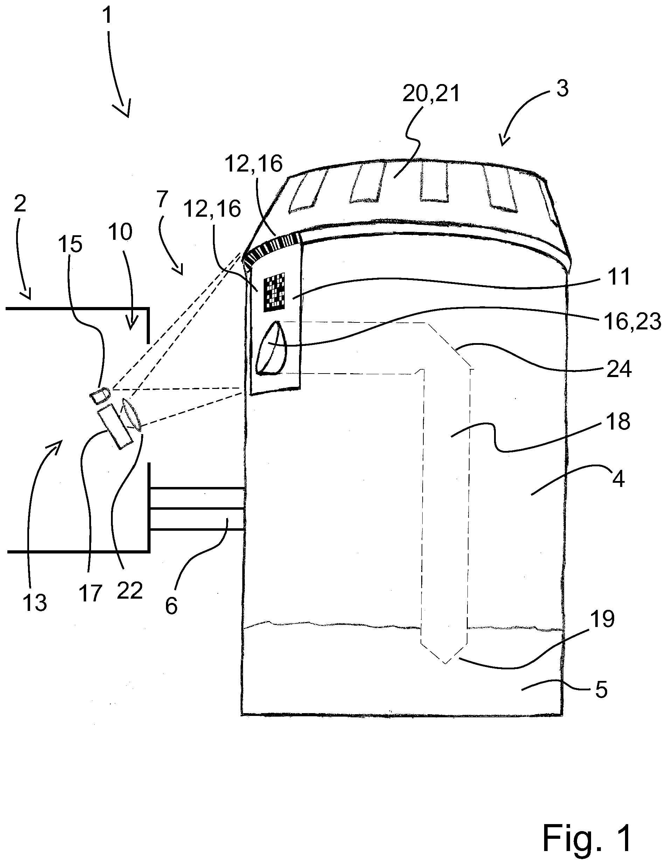

In Fig. 1 ist ein Anästhesiesystem 1 mit einem Anästhesiegerät 2 und einem Anästhesiemitteldosierer 3 dargestellt. Das Anästhesiesystem 1 dient dazu, bei der künstlichen Beatmung von Patienten insbesondere während chirurgischer Eingriffe ein Beatmungsgas mit Anästhesiemittel 5 anzureichern und dem Patienten zuzuführen. Der Anästhesiemitteldosierer 3 ist mittels mechanischer Befestigungseinrichtungen an dem Anästhesiegerät 2 befestigt. Die mechanischen Befestigungseinrichtungen sind in Fig. 1 nicht dargestellt. In dem Anästhesiemitteldosierer 3 ist ein Anästhesiemittel-Vorratsbehälter 4 mit dem Anästhesiemittel 5 vorhanden. Eine pneumatische Schnittstelle 6 dient zur pneumatischen Verbindung des Anästhesiemitteldosierers 3 mit dem Anästhesiegerät 2. Beatmungsgas kann damit aus dem Anästhesiegerät 2 durch den Anästhesiemittel-Vorratsbehälter 4 und anschließend wieder zurück in das Anästhesiegerät 2 geleitet werden. Während des Durchleitens des Beatmungsgases durch den Anästhesiemittel-Vorratsbehälter 4 wird das Beatmungsgas mit Anästhesiemittel 5 angereichert. Das Anästhesiemittel 5 verdunstet oder verdampft dabei in dem Anästhesiemittel-Vorratsbehälter 4.

Zur Steuerung der dem Beatmungsgas zuzuführenden Menge an dampfförmigen Anästhesiemittel 5 dient eine als Handrad 21 ausgebildete Stelleinrichtung 20. Das Handrad 21 kann dabei zur Steuerung der Menge an zuzuführenden Anästhesiemittel 5 zum Beatmungsgas manuell von Hand bewegt werden. Der Anästhesiemitteldosierer 3 weist hierbei einen Bypasskanal (nicht dargestellt) auf, durch welche das durch den Anästhesiemitteldosierer 3 geleitete Beatmungsgas an dem Anästhesiemittel-Vorratsbehälter 4 vorbeigeleitet werden kann. Mittels des Handrades 21 kann die Menge an Beatmungsgas eingestellt bzw. gesteuert werden, welches durch diesen Bypasskanal strömt. Je mehr Beatmungsgas durch den Bypasskanal geleitet wird, desto weniger Beatmungsgas strömt durch den Anästhesiemittel-Vorratsbehälter 4, desto weniger Anästhesiemittel 5 wird dem Beatmungsgas in dem Anästhesiemitteldosierer 3 zugeführt und umgekehrt (nicht dargestellt).

Der Anästhesiemitteldosierer 3 ist ein passiver Anästhesiemitteldosierer 3 und verfügt damit nicht über eine elektrische Heizeinrichtung zur Steuerung und/oder Regelung der Temperatur des Anästhesiemittels 5. Aus diesem Grund besteht zwischen dem Anästhesiegerät 2 und dem Anästhesiemitteldosierer 3 auch keine elektrische Verbindung zur Versorgung der nicht vorhandenen elektrischen Heizeinrichtung.

Das Anästhesiesystem 1 weist eine Parametererfassungseinrichtung 7 zur Übermittlung und Erfassung von Daten von dem Anästhesiemitteldosierer 3 zu dem Anästhesiegerät 2 auf. Die Parametererfassungseinrichtung 7 besteht aus einer Geräte-Schnittstelleneinheit 10 und einer Dosierer-Schnittstelleneinheit 11. Die Geräte-Schnittstelleneinheit 10 ist an dem Anästhesiegerät 2 und die Dosierer-Schnittstelleneinheit 11 ist an dem Anästhesiemitteldosierer 3 angeordnet. Die Geräte-Schnittstelleneinheit 10 umfasst wenigstens eine Kamera 17. Der Kamera 17 ist ferner eine Auswerteinheit nachgeschaltet (nicht dargestellt), die die Signale der Kamera 17 weiterverarbeitet. Die Dosierer-Schnittstelleneinheit 11 umfasst wenigstens ein Bildmuster 16.

Das im Wesentlichen tellerförmige Handrad 21 weist am gesamten äußeren Rand eine ringförmige optische Codierung 12 als Strichcode auf. Die optische Codierung 12 stellt das Bildmuster 16 der Dosierer-Schnittstelleneinheit 11 dar. Am Anästhesiegerät 2 ist im Bereich dieser optischen Codierung 12 des Handrades 21 ein optischer Encoder 13 angeordnet. Der optische Encoder 13 umfasst eine LED 15 und die Kamera 17. Die LED 15 emittiert Licht, das zu dem Bildmuster 16 umfassend die optischen Codierung 12 an der Außenseite des Handrades 21 gestrahlt wird. Aus dem von dem Bildmuster 16 reflektierten Licht kann die Kamera 17 die Codierung 12 des Bildmusters 16 erfassen und die Auswerteinheit im Anästhesiegerät 2 die Position des Handrades 21 ermitteln. Damit können dem Anästhesiegerät 2 Daten zur Stellung, insbesondere Winkelstellung des Handrades 21 zugeführt werden. Somit kann das Anästhesiegerät 2 Daten zur Konzentration an Anästhesiemittel des durch den Anästhesiemitteldosierer 3 geleiteten Beatmungsgases erhalten. Der optische Encoder 13 stellt ferner die Geräte-Schnittstelleneinheit 10 dar.

Unterhalb des Handrades 21 ist ein weiteres Bildmuster 16 angeordnet. Das Bildmuster 16 ist als optische Codierung 12 ausgeführt. Die optische Codierung 12 ist als Datamatrixcode angeordnet. Der Datamatrixcode beinhaltet Daten hinsichtlich der Art, der Sachnummer und der Seriennummer des Anästhesiemitteldosierers 3. Das Bildmuster 16 der optischen Codierung 12 kann durch die optische Encodereinheit 13 an dem Anästhesiegerät 2 erfasst werden. Die Kamera 17 erfasst dabei die in dem Bildmuster 16 der optischen Codierung 12 hinterlegten Daten.

Die optische Encodereinheit 13 zur Erfassung der Winkelposition des Handrades 21 mittels dem zugehörigen Bildmuster 16 der optische Codierung 12 auf dem Handrad 21 ist somit eine Parametererfassungseinrichtung 7 zur Erfassung einer Stellung der Stelleinrichtung 20 als Parameter des Anästhesiemitteldosierers 3.

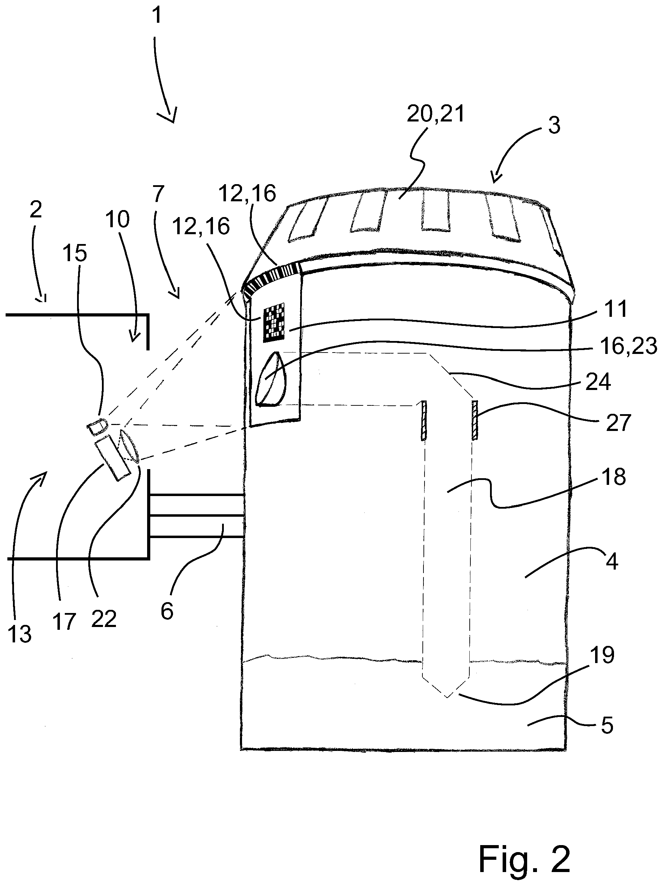

Das Anästhesiesystem 1 ist ferner mit einer Parametererfassungseinrichtung 7 zur Erfassung des Füllstandes des Anästhesiemittels 5 in dem Anästhesiemittel-Vorratsbehälter 4 versehen (Fig. 1, 2 und 3).

In einer ersten Ausführung, dargestellt in Fig. 1 trifft das von einer LED 15 emittierte Licht auf ein äußeres Einkoppelfenster 23 eines Lichtleiters 18 an der Dosierer-Schnittstelleneinheit 11. Der Lichtleiter 18 besteht aus Glas oder Kunststoff. Der Lichtleiter 18 ist L-förmig ausgebildet und weist einen im Wesentlichen horizontal und vertikal ausgerichteten Stab auf. An dem Einkoppelfenster 23 wird das von der LED 15 emittierte Licht in den Lichtleiter 18 eingekoppelt. Dieses in den horizontalen Stab des Lichtleiters 18 eingekoppelte Licht wird an einer Reflexionsfläche 24 des Lichtleiters 18 reflektiert und dadurch vertikal nach unten in den vertikalen Stab des Lichtleiters 18 geleitet. Dieses vertikal nach unten geleitete Licht gelangt dabei auf eine optische Grenzfläche 19. Die optische Grenzfläche 19 kann beispielsweise als ein 90° Konus ausgebildet sein. An der optischen Grenzfläche 19 treten in Abhängigkeit davon, ob die optische Grenzfläche 19 in das flüssige Anästhesiemittel 5 eingetaucht ist oder nicht unterschiedliche Berechungsindizes auf. Befindet sich die optische Grenzfläche 19 nicht in dem Anästhesiemittel 5, wird das zu der Grenzfläche 19 vertikal nach unten geleitete Licht im Wesentlichen vollständig als Totalreflexion an der optischen Grenzfläche 19 reflektiert und wieder vertikal nach oben in dem vertikalen Stab des Lichtleiters 18 geleitet. Dieses total reflektierte Licht wird anschließend an der Reflexionsfläche 24 reflektiert und dabei horizontal in dem horizontalen Stab des Lichtleiters 18 wieder zurück zu dem Einkoppelfenster 23 geleitet. An dem Einkoppelfenster 23 wird dieses an der optischen Grenzfläche total reflektierte Licht in einer Schräglage aufgrund des Brechungsindexunterschiedes nach unten zu einer Linse 22 und anschließend zu der Kamera 17 geleitet. Bei einem unterschiedlichen Füllstand an Anästhesiemittel 5 tritt ein unterschiedlicher Brechungsindex und somit eine unterschiedliche Strahlstärke des Lichtes auf, wobei das Bildmuster 16 aus dem Unterschied der Strahlstärke des Lichtes erzeugt wird. Die Kamera 17 als Empfänger von Licht kann dabei das Bildmuster 16 (die Strahlstärke dieses reflektierten Lichtes) erfassen. Befindet sich die optische Grenzfläche in dem flüssigen Anästhesiemittel, verringert sich der Brechungsindexunterschied der optischen Grenzfläche 19 so weit, dass der Totalreflexionswinkel im Wesentlichen überschritten wird und ein Großteil der elektromagnetischen Strahlung aus dem Lichtleiter 19 ausgekoppelt wird. Dadurch wird nur noch eine sehr geringe Menge an Licht an der optischen Grenzfläche reflektiert, so dass an der Kamera 17 nur eine sehr geringe Strahlstärke an reflektiertem Licht vorhanden ist. Damit kann der Füllstand des Anästhesiemittels 5 in dem Anästhesiemittel-Vorratsbehälter 4 erfasst werden. Bei einer hohen Strahlstärke des in die Kamera 17 geleiteten Lichtes befindet sich somit das Anästhesiemittel 5 in dem Anästhesiemittel-Vorratsbehälter 4 unterhalb der optischen Grenzfläche 19 des Lichtleiters 18, d. h. es ist nur noch eine sehr geringe Restmenge an Anästhesiemittel 5 in dem Anästhesiemittel-Vorratsbehälter 4 vorhanden. Bei einer geringen Strahlstärke an der Kamera 17 bei einer eingeschalteten LED befindet sich der Flüssigkeitsstand des Anästhesiemittels 5 in dem Anästhesiemittel-Vorratsbehälter 4 oberhalb der optischen Grenzfläche 19 des Lichtleiters 18. Diese Daten zum Füllstand können dabei von der Kamera 17 erfasst werden, die an dem Anästhesiegerät 2 angeordnet ist.

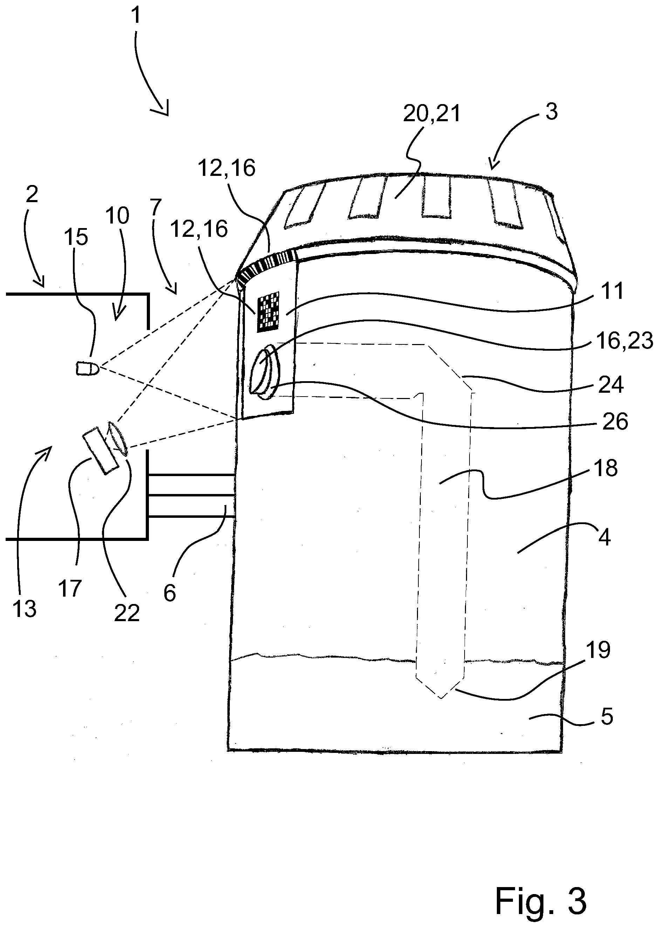

Alternativ dazu kann, wie in Fig. 3 dargestellt, das Einkoppelfenster 23 mit einem radial umlaufenden Kragen 26 ausgeführt sein. Die LED 15 ist dabei in Längsachse zu dem horizontalen Teil des Lichtleiters 18 angeordnet. Der Kragen 26 ermöglicht die Einkopplung des von der LED 15 emittierten Lichtes.

In einer weiteren Variante der Einrichtung zur Erfassung des Füllstandes 7 ist der obere vertikale Teil des Lichtleiters 18 von einer Hülse 27 aus reflektierendem Material umgeben. Damit kann ein bleibender Ring in dem Bildmuster 16 und damit im Sichtfeldbereich der Kamera 17 erzeugt werden, der die Position des vertikalen Teils des Lichtleiters 18 im Sichtfeldbereich der Kamera 17 markiert. In vorteilhafter Weise kann somit der Detektionsbereich für den Helligkeitsumschlag auch bei einem voll gefüllten Anästesiemittlel-Vorratsbehälters 4 festgelegt werden. Ferner kann anhand der Lichtintensität des Ringes eine Funktionsfähigkeit der Schnittstelle zwischen horizontalem und vertikalen Teils des Lichtleiters 18 erkannt werden.

Vorzugsweise wird von der LED 15 elektromagnetische Strahlung als für den Menschen nicht sichtbare Infrarotstrahlung emittiert, so dass die optische Erfassung von Daten nicht sichtbar ist. Das Einkoppelfenster 23 des Lichtleiters 18 stellt dabei ebenfalls die Dosierer-Schnittstelleneineheit 11 dar, weil hier in analoger Weise zu einer optischen Codierung 12 eine Bildmuster 16 erzeugt werden kann und damit Daten von dem Anästhesiemitteldosierer an das Anästhesiegerät übermittelt werden können.

Insgesamt betrachtet sind mit dem erfindungsgemäßen Anästhesiesystem 1 erhebliche Vorteile verbunden. Die Übermittlung von Daten von dem Anästhesiemitteldosierer 3 zu dem Anästhesiegerät 2 erfolgt einfach und zuverlässig mittels optischer Einrichtungen. Dabei ist es nicht erforderlich, dass der Anästhesiemitteldosierer 3 mit Energie zum Betrieb einer Parametererfassungseinrichtung 7 des Anästhesiemitteldosierers 3 versorgt wird. Der Anästhesiemitteldosierer 3 kann damit konstruktiv einfach und preiswert in der Herstellung, insbesondere als passiver Anästhesiemitteldosierer 3, ausgeführt werden.

### BEZUGSZEICHENLISTE

- 1: Anästhesiesystem
- 2: Anästhesiegerät
- 3: Anästhesiemitteldosierrer
- 4: Anästhesiemittel-Vorratsbehälter
- 5: Anästhesiemittel
- 6: Pneumatische Schnittstelle
- 7: Parametererfassungseinrichtung
- 10: Geräte-Schnittstelleneinheit
- 11: Dosierer-Schnittstelleneinheit
- 12: Optische Codierung
- 13: Optischer Encoder
- 15: LED
- 16: Bildmuster
- 17: Kamera
- 18: Lichtleiter
- 19: Optische Grenzfläche
- 20: Stelleinrichtung
- 21: Handrad
- 22: Linse
- 23: Einkoppelfenster
- 24: Reflexionsfläche
- 26: Kragen
- 27: Hülse

## Patentansprüche

1. Anästhesiesystem (1), umfassend
ein Anästhesiegerät (2),
wenigstens einen Anästhesiemitteldosierer (3) wenigstens eine Parametererfassungseinrichtung (7) zur Erfassung wenigstens zweier Parameter des wenigstens einen Anästhesiemitteldosierers (3) an dem Anästhesiegerät (2),
wobei die Parametererfassungseinrichtung (7) mit einer Geräte-Schnittstelleneinheit (10) mit wenigstens einer Kamera (17) an dem Anästhesiegerät (2) und
einer Dosierer-Schnittstelleneinheit (11) mit wenigstens zwei Bildmustern (16) an dem wenigstens einen Anästhesiemitteldosierer (3) ausgebildet ist, wobei die wenigstens zwei Bildmuster (16) von der Kamera (17) erfassbar sind und die Dosierer-Schnittstelleneinheit (11) eine passive Dosierer-Schnittstelleneinheit (11) ist, wobei mit der wenigstens einen Parametererfassungseinrichtung (7) mittels eines der wenigstens zwei Bildmuster (16) ein Füllstand an Anästhesiemittel (5) des Anästhesiemitteldosierers (3) an dem Anästhesiegerät (2) erfassbar ist.

2. Anästhesiesystem nach Anspruch 1, **dadurch gekennzeichnet, dass** die Übermittlung von Daten von dem wenigstens einen Anästhesiemitteldosierer (3) an das Anästhesiegerät (2) mittels Infrarotstrahlung oder sichtbares Licht oder UV-Strahlung ausführbar ist.

3. Anästhesiesystem nach einem der vorhergehenden Ansprüche, **dadurch gekennzeichnet, dass** die Kamera (17) im sichtbaren Spektralbereich oder infraroten Bereich oder im UV-Bereich arbeitet.

4. Anästhesiesystem nach einem der vorhergehenden Ansprüche, **dadurch gekennzeichnet, dass** das wenigstens eine Bildmuster (16) der Dosierer-Schnittstelleneinheit (11) eine Codierung (12) umfasst.

5. Anästhesiesystem nach Anspruch 4, **dadurch gekennzeichnet, dass** die Codierung (12) eine optische Codierung (12) ist.

6. Anästhesiesystem nach Anspruch 5, **dadurch gekennzeichnet, dass** die optische Codierung (12) ein Strichcode oder ein zweidimensionaler Code, vorzugsweise ein Datamatrixcode ist.

7. Anästhesiesystem nach einem der vorhergehenden Ansprüche, **dadurch gekennzeichnet, dass** das wenigstens eine Bildmuster (16) Daten bezüglich der Art des Anästhesiemitteldosierers (3) und/oder einer Sachnummer des Anästhesiemitteldosierers (3) und/oder einer Seriennummer des Anästhesiemitteldosierers (3) und/oder der Art des Anästhesiemittels (5) umfasst.

8. Anästhesiesystem nach Anspruch 5, **dadurch gekennzeichnet, dass** die Geräte-Schnittstelleneinheit (10) einen optischen Encoder (13) zur optischen Erfassung der optischen Codierung (12) der Dosierer-Schnittstelleneinheit (11) umfasst.

9. Anästhesiesystem nach Anspruch 8, **dadurch gekennzeichnet, dass** der optische Encoder (13) aus einer LED (15) und der Kamera (17) besteht.

10. Anästhesiesystem nach einem oder mehreren der vorhergehenden Ansprüche, **dadurch gekennzeichnet, dass** der Füllstand an Anästhesiemittel (5) mittels einer Veränderung eines Brechungsindexverhältnisses einer elektromagnetischen Strahlung ermittelbar ist, wobei bei einem unterschiedlichen Füllstand ein unterschiedlicher Brechungsindex und somit eine unterschiedliche Strahlstärke der elektromagnetischen Strahlung auftritt, wobei das wenigstens eine Bildmuster (16) aus dem Unterschied der Strahlstärke der elektromagnetischen Strahlung erzeugt wird.

11. Anästhesiesystem nach einem der Ansprüche 8 bis 10,
**dadurch gekennzeichnet, dass** von dem optischen Encoder (13) Licht in einen Lichtleiter (18) der Dosierer-Schnittstelleneinheit (11) einleitbar ist, wobei das eingeleitete Licht an einer optischen Grenzfläche (19) in Abhängigkeit von dem Füllstand des Anästhesiemittels (5) reflektierbar ist und von der Strahlstärke des reflektierten Lichtes das Bildmusters (16) erzeugt wird, welches von der Kamera (17) erfassbar ist, wobei die Strahlstärke ein Maß für den Füllstand an Anästhesiemittel (5) des Anästhesiemitteldosierers (3) ist.

12. Anästhesiesystem nach Anspruch 11, **dadurch gekennzeichnet, dass** ein Teil des Lichtleiters (18) von einer Hülse (27) aus reflektierenden Material umgeben ist.

13. Anästhesiesystem nach einem oder mehreren der vorhergehenden Ansprüche, **dadurch gekennzeichnet, dass** mit der wenigstens einen Parametererfassungseinrichtung (7) eine Stellung, insbesondere Winkelstellung, einer Stelleinrichtung (20), insbesondere eines Handrades (21) des Anästhesiemitteldosierers (3) an dem Anästhesiegerät (2) erfassbar ist.

14. Anästhesiesystem nach Anspruch 13, **dadurch gekennzeichnet, dass** an der Stelleinrichtung (20) ein Bildmuster (16) ausgebildet ist, welches von der Kamera (17) zur Erfassung der Stellung der Stelleinrichtung (20) erfassbar ist.

## Claims

1. Anaesthetic system (1), comprising
an anaesthesia apparatus (2),
at least one anaesthetic dispenser (3),
at least one parameter detection device (7) for detecting at least two parameters of the at least one anaesthetic dispenser (3) on the anaesthesia apparatus (2),
wherein the parameter detection device (7) is formed with an apparatus interface unit (10) having at least one camera (17) on the anaesthesia apparatus (2) and a dispenser interface unit (11) having at least two image patterns (16) on the at least one anaesthetic dispenser (3), wherein the at least two image patterns (16) can be detected by the camera (17) and the dispenser interface unit (11) is a passive dispenser interface unit (11), wherein, by using the at least one parameter detection device (7), a filling level of anaesthetic (5) of the anaesthetic dispenser (3) on the anaesthesia apparatus (2) can be detected by means of one of the at least two image patterns (16).

2. Anaesthetic system according to Claim 1, **characterized in that** the transmission of data from the at least one anaesthetic dispenser (3) on the anaesthesia apparatus (2) can be carried out by means of infrared radiation or visible light or UV radiation.

3. Anaesthetic system according to one of the preceding claims, **characterized in that** the camera (17) operates in the visible spectral range or infrared range or in the UV range.

4. Anaesthetic system according to one of the preceding claims, **characterized in that** the at least one image pattern (16) of the dispenser interface unit (11) comprises a code (12).

5. Anaesthetic system according to Claim 4, **characterized in that** the code (12) is an optical code (12).

6. Anaesthetic system according to Claim 5, **characterized in that** the optical code (12) is a bar code or a two-dimensional code, preferably a data matrix code.

7. Anaesthetic system according to one of the preceding claims, **characterized in that** the at least one image pattern (16) comprises data relating to the type of anaesthetic dispenser (3) and/or a part number of the anaesthetic dispenser (3) and/or a serial number of the anaesthetic dispenser (3) and/or the type of anaesthetic (5).

8. Anaesthetic system according to Claim 5, **characterized in that** the apparatus interface unit (10) comprises an optical encoder (13) for the optical detection of the optical code (12) of the dispenser interface unit (11).

9. Anaesthetic system according to Claim 8, **characterized in that** the optical encoder (13) is composed of an LED (15) and the camera (17).

10. Anaesthetic system according to one or more of the preceding claims, **characterized in that** the filling level of anaesthetic (5) can be determined by means of a change in the refractive index ratio of an electromagnetic radiation, wherein, in the event of a different filling level, a different refractive index and thus a different radiant intensity of the electromagnetic radiation occurs, wherein the at least one image pattern (16) is generated from the difference in the radiant intensity of the electromagnetic radiation.

11. Anaesthetic system according to one of Claims 8 to 10, **characterized in that** light from the optical encoder (13) can be introduced into an optical waveguide (18) of the dispenser interface unit (11), wherein the light introduced can be reflected at an optical interface (19), depending on the filling level of the anaesthetic (5), and the image pattern (16) is generated from the radiant intensity of the reflected light and can be detected by the camera (17), wherein the radiant intensity is a measure of the filling level of the anaesthetic (5) of the anaesthetic dispenser (3).

12. Anaesthetic system according to Claim 11, **characterized in that** part of the optical waveguide (18) is surrounded by a sleeve (27) of reflective material.

13. Anaesthetic system according to one or more of the preceding claims, **characterized in that** by using the at least one parameter detection device (7), a position, in particular an angular position, of an actuating device (20), in particular a hand wheel (21) of the anaesthetic dispenser (3) on the anaesthesia apparatus (2), can be detected.

14. Anaesthetic system according to Claim 13, **characterized in that** an image pattern (16), which can be detected by the camera (17) for detecting the position of the actuating device (20), is formed on the actuating device (20).

## Revendications

1. Système d'anesthésie (10), comprenant
un appareil d'anesthésie (2),
au moins un dispositif de dosage d'agent anesthésique (3),
au moins un dispositif de détection de paramètres (7) destiné à détecter au moins deux paramètres dudit au moins un dispositif de dosage d'agent anesthésique (3) de l'appareil d'anesthésie (2),
dans lequel le dispositif de détection de paramètres (7) est constitué d'une unité d'interface d'appareil (10) comportant au moins une caméra (17) sur l'appareil d'anesthésie (2) et
une unité d'interface de doseur (11) comportant au moins deux motifs d'image (16) sur ledit au moins dispositif de dosage d'agent anesthésique (3), dans lequel lesdits au moins deux motifs d'image (16) peuvent être détectés par la caméra (17) et l'unité d'interface de doseur (11) est une unité d'interface de doseur (11), passive, dans lequel un niveau de remplissage d'agent anesthésique (5) du dispositif de dosage d'agent anesthésique (3) peut être détecté sur l'appareil anesthésique (2) à l'aide dudit au moins un dispositif de détection de paramètres (7) et au moyen de l'un desdits au moins deux motifs d'images (16).

2. Système d'anesthésie selon la revendication 1, **caractérisé en ce que** la transmission de données dudit au moins un dispositif de dosage d'agent anesthésique (3) à l'appareil anesthésique (2) peut être effectuée au moyen d'un rayonnement infrarouge ou de lumière visible ou d'un rayonnement UV.

3. Système d'anesthésie selon l'une des revendications précédentes, **caractérisé en ce que** la caméra (17) fonctionne dans le domaine spectral visible ou dans le domaine infrarouge ou dans le domaine UV.

4. Système d'anesthésie selon l'une des revendications précédentes, **caractérisé en ce que** ledit au moins un motif d'image (16) de l'unité d'interface de doseur (11) comprend un codage (12).

5. Système d'anesthésie selon la revendication 4, **caractérisé en ce que** le codage (12) est un codage optique.

6. Système d'anesthésie selon la revendication 5, **caractérisé en ce que** le codage optique (12) est un code à barres ou un code bidimensionnel, de préférence un code de type Datamatrix.

7. Système d'anesthésie selon l'une des revendications précédentes, **caractérisé en ce que** ledit au moins un motif d'image (16) comprend des données relatives au type de dispositif de dosage d'agent anesthésique (3) et/ou à un numéro de pièce du dispositif de dosage d'agent anesthésique (3) et/ou à un numéro de série du dispositif de dosage d'agent anesthésique (3) et/ou au type de l'agent anesthésique (5).

8. Système d'anesthésie selon la revendication 5, **caractérisé en ce que** l'unité d'interface d'appareil (10) comprend un codeur optique (13) destiné à détecter optiquement le codage optique (12) de l'unité d'interface de doseur (11).

9. Système d'anesthésie selon la revendication 8, **caractérisé en ce que** le codeur optique (13) est constitué d'une LED (15) et de la caméra (17).

10. Système d'anesthésie selon l'une ou plusieurs des revendications précédentes, **caractérisé en ce que** le niveau de remplissage d'agent anesthésique (5) peut être déterminé au moyen d'une modification d'un indice de réfraction du rayonnement électromagnétique, dans lequel, dans le cas d'un niveau de remplissage différent, un indice de réfraction différent et par conséquent, une intensité de rayonnement différente du rayonnement électromagnétique se produit, dans lequel ledit au moins un motif d'image (16) est généré à partir de la différence d'intensité de rayonnement du rayonnement électromagnétique.

11. Système d'anesthésie selon l'une des revendications 8 à 10, **caractérisé en ce que** la lumière provenant du codeur optique (13) peut être introduite dans un guide de lumière (18) de l'unité d'interface de doseur (11), dans lequel la lumière introduite peut être réfléchie sur une interface optique (19) en fonction du niveau de remplissage de l'agent anesthésique (5) et **en ce que** le motif d'image (16) est généré à partir de l'intensité de rayonnement de la lumière réfléchie et peut être détecté par la caméra (17), dans lequel l'intensité de rayonnement est une mesure du niveau de remplissage d'agent anesthésique (5) dans le dispositif de dosage d'agent anesthésique (3).

12. Système d'anesthésie selon la revendication 11, **caractérisé en ce qu'**une partie du guide de lumière (18) est entourée d'un manchon (27) en matériau réfléchissant.

13. Système d'anesthésie selon l'une ou plusieurs des revendications précédentes, **caractérisé en ce qu'**un réglage, notamment un réglage angulaire, d'un dispositif de réglage (20), notamment d'une molette (21) du dispositif de dosage d'agent anesthésique (3), peut être détecté sur l'appareil d'anesthésie (2) à l'aide dudit au moins un dispositif de détection de paramètres (7).

14. Système d'anesthésie selon la revendication 13, **caractérisé en ce qu'**un motif d'image (16) qui peut être détecté par la caméra (17) afin de détecter le réglage du dispositif de réglage (20) est réalisé sur le dispositif de réglage (20).
